# EUROPEAN PATENT APPLICATION

(11) **EP 3 907 688 A1**
(43) Date of publication of application: **10.11.2021**
(21) Application number: 19922193.8
(22) Date of filing: 26.03.2019
(51) Int. Cl.: G06Q 30/06

(54) **INFORMATION PROCESSING DEVICE, INFORMATION PROCESSING METHOD, AND PROGRAM**

(71) Applicant: Japan Tobacco Inc., Tokyo 105-6927 (JP)
(72) Inventor: SERITA, Kazutoshi, Tokyo 130-8603 (JP); SENJU, Masatoshi, Tokyo 130-8603 (JP); ITABASHI, Masato, Tokyo 130-8603 (JP)
(74) Representative: Hoffmann Eitle
(86) International application number: PCT/JP2019/012911
(87) International publication number: WO 2020/194518

(57) **Abstract**

An information processing device comprising: an acquisition unit 311 that obtains smoking information from a flavor inhaler, etc., 1 associated to a user terminal 2; a creation unit 313 that creates output information for outputting a message on the basis of the smoking information, to the user terminal 2; and a transmission unit 315 that sends the output information to the user terminal 2 or a control device that controls the output of messages to the user terminal 2. As a result, the retention rate for users using the flavor inhaler can be increased.

## Description

### Technical Field

The present invention relates to an information processing device, an information processing method, and a program.

### Background Art

In these years, techniques for providing various pieces of information for users of flavor inhalers using communication tools are being developed. With respect to this, in PTL 1, a technique for notifying, when a user is using a flavor inhaler, the user of information regarding a conversation with another user or an advertisement using an instant messaging service on the basis of whether the user can have a conversation is disclosed. In addition, in PTL 2, a technique for communicating favorite information or recommendation information between users of flavor inhalers using a social network including the users is disclosed.

### Citation List

### Patent Literature

PTL 1: U.S. Patent Application Publication No. 2016/0285983
PTL 2: U.S. Patent Application Publication No. 2016/0337444

### Summary of Invention

### Technical Problem

With the conventional techniques described in PTLs 1 and 2, a message regarding a flavor inhaler can be provided for a user of the flavor inhaler. With these conventional techniques, however, a message whose content is determined in advance is provided or the user just creates a message that he/she desires by operating a terminal apparatus. Since appropriate information cannot be provided in accordance with a smoking state of the user, therefore, a retention rate for users using a flavor inhaler or an apparatus including a mouthpiece for cigarettes (hereinafter referred to as a "flavor inhaler etc.") might undesirably decrease.

Some aspects of the present invention have been conceived in view of such circumstances and aim to provided a technique capable of increasing the retention rate for users using a flavor inhaler etc.

### Solution to Problem

An information processing device according to an aspect of the present invention includes an acquisition unit that obtains smoking information from a flavor inhaler associated to a user terminal, a creation unit that creates output information for outputting a message based on the smoking information to the user terminal, and a transmission unit that sends the output information to the user terminal or a control device that controls output of messages to the user terminal.

An information processing method according to another aspect of the present invention is an information processing method executed by a computer. The method includes the steps of obtaining smoking information from a flavor inhaler associated to a user terminal, creating output information for outputting a message based on the smoking information to the user terminal, and sending the output information to the user terminal or a control device that controls output of messages to the user terminal.

A program according to another aspect of the present invention causes a computer to achieve an acquisition unit that obtains smoking information from a flavor inhaler associated to a user terminal, a creation unit that creates output information for outputting a message based on the smoking information to the user terminal, and a transmission unit that sends the output information to the user terminal or a control device that controls output of messages to the user terminal.

According to these aspects, output information for outputting a message based on smoking information from a flavor inhaler to a user terminal is created and sent to the user terminal or a control device that controls the output of messages to the user terminal. Since output information can be appropriately provided in accordance with a smoking state of the user, therefore, a retention rate for users using a flavor inhaler can be increased.

In the present invention, "unit" and "device" do not just refer to physical means but also refer to a case where functions of "unit" and "device" are achieved by software. A function of a single "unit" or "device" may be achieved by two or more physical means or devices, or functions of two or more "units" or "devices" may be achieved by single physical means or device. Advantageous Effects of Invention

According to the present invention, a retention rate for users using a flavor inhaler can be increased.

### Brief Description of Drawings

[Fig. 1] Fig. 1 is a schematic configuration diagram of an output information provision system according to a first embodiment of the present invention.
[Fig. 2A] Fig. 2A is a block diagram illustrating a schematic configuration of a flavor inhaler according to the first embodiment of the present invention.
[Fig. 2B] Fig. 2B is a diagram illustrating an example of a schematic appearance of the flavor inhaler according to the first embodiment of the present invention.
[Fig. 3] Fig. 3 is a block diagram illustrating a schematic configuration of the flavor inhaler according to the first embodiment of the present invention.
[Fig. 4] Fig. 4 is a block diagram illustrating another schematic configuration of the flavor inhaler according to the first embodiment of the present invention.
[Fig. 5] Fig. 5 is a diagram illustrating another example of the schematic appearance of the flavor inhaler according to the first embodiment of the present invention.
[Fig. 6] Fig. 6 illustrates another example of the schematic appearance of the flavor inhaler at a time when the flavor inhaler holds an aerosol-source material according to the first embodiment of the present invention.
[Fig. 7] Fig. 7 is a block diagram illustrating a schematic configuration of a user terminal according to the first embodiment of the present invention.
[Fig. 8] Fig. 8 is a schematic configuration diagram illustrating an example of a functional configuration of an information processing server according to the first embodiment of the present invention.
[Fig. 9] Fig. 9 is a flowchart illustrating an example of a process for sending output information according to the first embodiment of the present invention.
[Fig. 10] Fig. 10 is a conceptual diagram illustrating an example of the process for sending output information according to the first embodiment of the present invention.
[Fig. 11] Fig. 11 is a diagram illustrating an example of a screen output on the user terminal according to the first embodiment of the present invention.
[Fig. 12] Fig. 12 is a schematic configuration diagram of an output information provision system according to a second embodiment of the present invention.
[Fig. 13] Fig. 13 is a diagram illustrating an example of a screen output on a user terminal according to the second embodiment of the present invention.
[Fig. 14] Fig. 14 is a schematic configuration diagram of an output information provision system according to a third embodiment of the present invention.
[Fig. 15] Fig. 15 is a diagram illustrating an example of screens output on user terminals according to the third embodiment of the present invention.
[Fig. 16] Fig. 16 is a diagram illustrating an example of the hardware configuration of a computer according to an embodiment of the present invention.

### Description of Embodiments

Embodiments of the present invention will be described hereinafter with reference to the accompanying drawings. The following embodiments are examples for describing the present invention and not intended to limit the present invention thereto. In addition, the present invention can be modified in various ways insofar as the scope thereof is not deviated from. Furthermore, the same components are given the same reference numerals in the drawings as much as possible, and redundant description thereof is omitted.

### <First Embodiment>

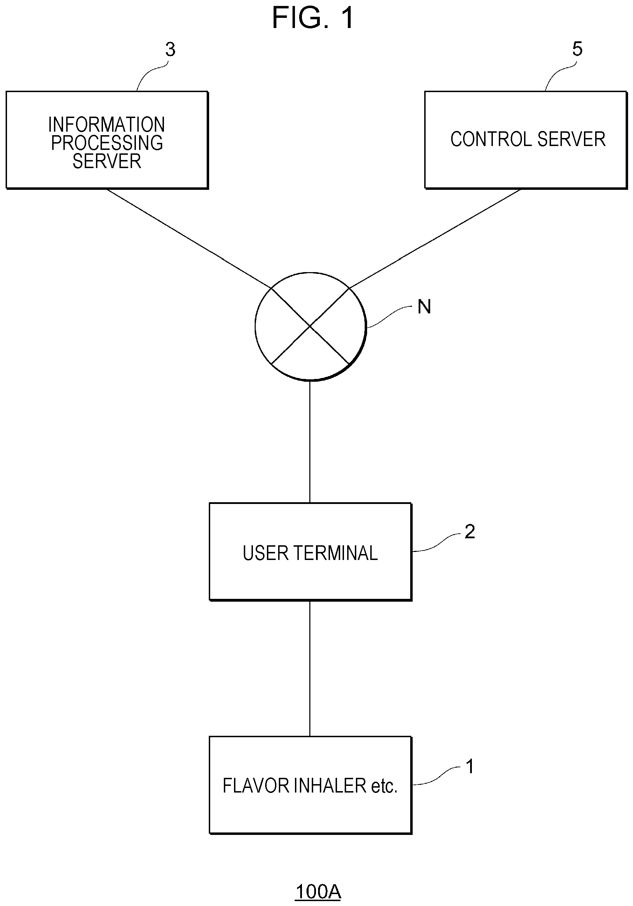

Fig. 1 is a schematic configuration diagram (system configuration diagram) of an output information provision system according to a first embodiment of the present invention. As illustrated in Fig. 1, an output information provision system 100A includes, for example, a flavor inhaler etc. 1 (flavor inhaler), a user terminal 2 used by a user, an information processing server 3 (information processing device) that creates output information for outputting a message based on smoking information to the user terminal 2 and that sends the output information to a control server 5, which will be described later, and the control server 5 (control device) that controls the output of messages based on smoking information to the user terminal 2.

Although the information processing server 3 and the control server 5 will be described in the following description as separate components in the present embodiment, the configuration of the information processing server 3 and the control server 5 is not limited to this. For example, the information processing server 3 and the control server 5 may be integrated with each other, instead. That is, the information processing server 3 has the configuration and functions of the control server 5, which will be described later. In this case, the information processing server 3 (control server 5) sends output information to the user terminal.

An "flavor inhaler etc." refers to an apparatus for inhaling a flavor and implies, but is not limited to, for example, an electronic cigarette, a heat-not-burn tobacco cigarette, or a conventional cigarette. The flavor inhaler includes an aerosol generation device for inhaling a generated aerosol. The aerosol generation device implies, for example, an electronic cigarette, a heat-not-burn tobacco cigarette, or a medical nebulizer. More specifically, the aerosol generation device is, for example, a device that generates an aerosol by atomizing a liquid (aerosol source) using electric power. The aerosol is obtained by atomizing the aerosol source and is particles fine enough to float in a gas. The aerosol generated by the aerosol generation device may have a flavor. Examples of the aerosol generation device include heat-not-burn tobacco cigarettes (T-vapor and Infused) and electronic cigarettes (E-vapor). The aerosol generation device may be of a type that directly heats tobacco (direct heating), a type that indirectly heats tobacco (indirect heating), or a type that heats a liquid. Alternatively, the aerosol generation device may atomize a liquid by generating SAWs (surface acoustic waves) using a piezoelectric substrate including a pair of comb electrodes.

The "message based on smoking information" (hereinafter referred to as a "message") is information based on "smoking information" from the flavor inhaler etc. 1 and output to the user terminal 2. The message is information with which a retention rate for users using the flavor inhaler etc. 1 can be increased. The message may include, for example, text information, audio information, movie information, or image information based on a smoking condition of a user or a use condition of the flavor inhaler etc. 1 used by a user. More specifically, the message may include advertisement information regarding smoking or the flavor inhaler etc. 1, failure information regarding the flavor inhaler etc. 1, a coupon indicating a discount for an attachment of the flavor inhaler etc. 1 or the like, PR movie information associated to a smoking area, or the like. The message may also include image information, movie information, audio information, or the like corresponding to a character, which will be described later. The message may also include a URL (uniform resource locator) of a certain website on the Internet, a QR code (registered trademark), or the like. The message may also include Morse code, a certain encrypted code, or the like.

The "smoking information" is information regarding smoking and includes, for example, the frequency of smoking, the amount of smoking, and facts of smoking. The smoking information is not limited to these and includes information regarding a use condition of the flavor inhaler etc. 1. The information regarding the use condition of the flavor inhaler etc. 1 includes, for example, information regarding the use frequency and a use period of the flavor inhaler etc. 1, a charging status or a remaining amount of a battery included in the flavor inhaler etc. 1, a position of the flavor inhaler etc. 1 (user), a fact that a certain capsule has been attached to the flavor inhaler etc. 1, a period for which the user has carried the flavor inhaler etc., a period for which the user has held the flavor inhaler etc., or the like. Alternatively, the smoking information may be, for example, information regarding the number of puffs, the number of times that refills have been replaced, the amount of liquid consumed, the remaining amount of liquid, or the like. The refills are replaceable members such as a reservoir, a flavor source, a smoking article 140, and various atomizers, which will be described later, included in the flavor inhaler etc. 1. The smoking information can be measured by various sensors and the like included in the flavor inhaler etc. 1, which will be described later.

The control server 5 is a system that performs a process for exchanging information over a network N. The control server 5 mediates information exchange between the information processing server 3 and the user terminal 2, for example, and facilitates a conversation between the user and the information processing server 3, such as a voice call or a chat employing text information. Stickers and movies that are image information for communication, for example, may be used for the process for exchanging information, as well as text information and audio information input over the network N. The control server 5 is thus a server computer that manages a conversation on the certain network N and achieves a server function when a certain server program operates on the server computer. Although the control server 5 includes an information exchange system in the present embodiment, the information processing server 3 may include the information exchange system, instead.

The network N is a communication link or a communication network relating to information processing, such as the Internet. A specific configuration of the network N is not particularly limited insofar as the user terminal 2, the information processing server 3, and the control server 5 can communicate data with one another.

The flavor inhaler etc. 1 and the user terminal 2 are associated to each other and become able to communicate data with each other by establishing short-distance wireless communication such as Bluetooth [registered trademark] or BLE (Bluetooth low energy) communication. The communication of data between the flavor inhaler etc. 1 and the user terminal 2 is not limited to these communication technologies, and may be achieved by any communication technology, such as Wi-Fi (registered trademark), LPWAN (low power wide-area network), or NFC (near-field communication). The communication of data between the flavor inhaler etc. 1 and the user terminal 2 is not necessarily limited to wireless communication, and may be wired communication achieved by, for example, USB (universal serial bus), mini-USB, micro-USB, or Lightning, instead.

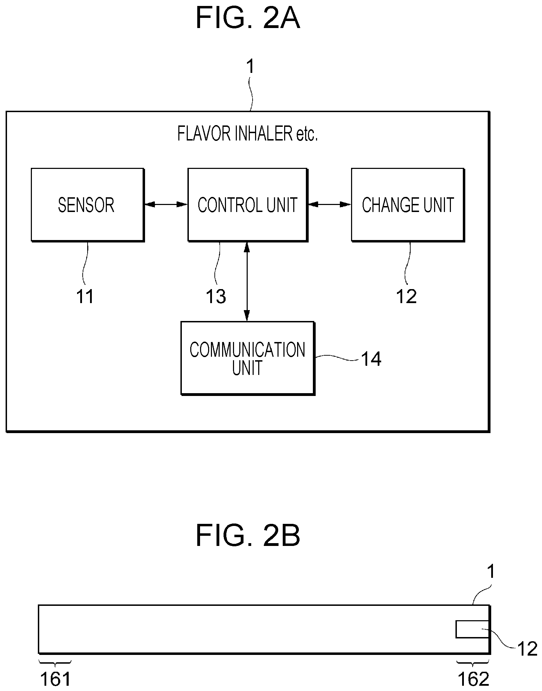

Fig. 2A is a block diagram of a schematic configuration of the flavor inhaler according to the first embodiment of the present invention. It is to be noted that Fig. 2A schematically and conceptually illustrates the components included in the flavor inhaler etc. 1 and does not illustrate strict positions, shapes, dimensions, positional relationships, or the like of the components and the flavor inhaler etc. 1. It is also to be noted that the flavor inhaler etc. 1 can include a component that is not illustrated in Fig. 2A, such as a tobacco capsule or a liquid cartridge.

A sensor 11 is, for example, a sensor for detecting an inhalation by the user. The sensor 11 may be a sensor of any type for detecting an inhalation by the user, such as a flow rate sensor, a flow velocity sensor, or a pressure sensor. The sensor 11 may be a button pressed by the user to inhale, instead. For example, the sensor 11 may be an inhalation sensor, instead, and detect an inhalation by the user using the flavor inhaler etc. 1. The sensor 11 may be an airflow sensor, instead, and detect an airflow caused by an inhalation by the user. The sensor 11 may be a GPS sensor for measuring a position of the flavor inhaler etc. 1, a gyro sensor for detecting an angle, an attitude, or the like of the flavor inhaler etc. 1, or the like, instead.

A change unit 12 is a block where a certain change that can be observed from the outside occurs. The change unit 12 may be an LED (light-emitting diode) that emits light of a certain color, such as a blue LED, and the certain change may be emission of light of a certain color, such as blue. The certain color is not limited to blue and may be any color. The certain change may be a change in the color of light emitted or the intensity of light emitted according to the strength of inhalation detected by the sensor 11. The change unit 12 is not limited to an LED and may be a light source of a different configuration that emits light of a certain color.

Fig. 2B is a diagram illustrating an example of a schematic appearance of the flavor inhaler according to the first embodiment of the present invention. The configuration of the change unit 12 will be described with reference to Fig. 2B. As illustrated in Fig. 2B, the flavor inhaler etc. 1 may have, but is not limited to, a shape of a stick including two ends 161 and 162. The user puts the end 161 in his/her mouth for inhalation. The user may put in his/her mouth a mouthpiece for cigarettes that can be attached to the end 161, instead, in order to inhale through the end 161. When the flavor inhaler etc. 1 has the shape illustrated in Fig. 2B, the change unit 12 is preferably provided at the other 162 of the two ends. The change unit 12 is at least a part of an outer surface of the flavor inhaler etc. 1 and may have any shape such as a substantially rectangular shape illustrated in Fig. 2B or a ring shape (not illustrated) around the circumference of the flavor inhaler etc. 1.

Fig. 2A will be referred to again. A control unit 13 is a block that at least causes a certain change in the change unit 12 on the basis of a signal from the sensor 11. For example, the control unit 13 may cause emission of light of a certain color in the change unit 12 if the strength of a signal from the sensor 11 or the strength of inhalation determined on the basis of the signal is higher than or equal to a certain threshold. In addition, for example, the control unit 13 may change the color or intensity of light emitted from the change unit 12 in accordance with the strength of a signal from the sensor 11 or the strength of inhalation determined on the basis of the signal. The control unit 13 may increase the intensity of light emitted when an inhalation is strong, for example, and decrease the intensity of light emitted when an inhalation is weak. Alternatively, for example, the control unit 13 may divide the strength of inhalation into plural stages, set a certain color for each of the stages, and cause emission of light of a certain color corresponding to the strength of inhalation. The control unit 13 may be an electronic circuit module configured as a microprocessor or a microcomputer.

A communication unit 14 communicates with the user terminal or another computer. The communication unit 14 may be achieved using at least a network interface or the like as a hardware resource. The control unit 13 is capable of sending various pieces of information detected by the sensor 1 1 to the user terminal or another computer via the communication unit 14. The information from various locations sent from the control unit 13 to the user terminal or another computer via the communication unit 14 is, for example, smoking information. The smoking information will be described later. The communication unit 14 is capable of receiving various pieces of information sent from the user terminal or another computer.

The flavor inhaler etc. 1 according to the embodiment of the present invention includes a conventional cigarette. A conventional cigarette does not include the sensor 11, the control unit 13, and the communication unit 14, but a burning part thereof corresponds to the change unit 12. This is because a burning part of a conventional cigarette causes certain changes in color and temperature on the basis of an inhalation by the user.

When the flavor inhaler etc. 1 is a conventional cigarette (e.g., a cigarette), a mouthpiece attached to an end of the cigarette (e.g., a mouthpiece for cigarettes) may include some or all of the functions of the sensor 11, the control unit 13, and the communication unit 14 illustrated in Fig. 2A. With this configuration, the mouthpiece can send various pieces of information detected by the sensor, such as smoking information, to the user terminal or another computer.

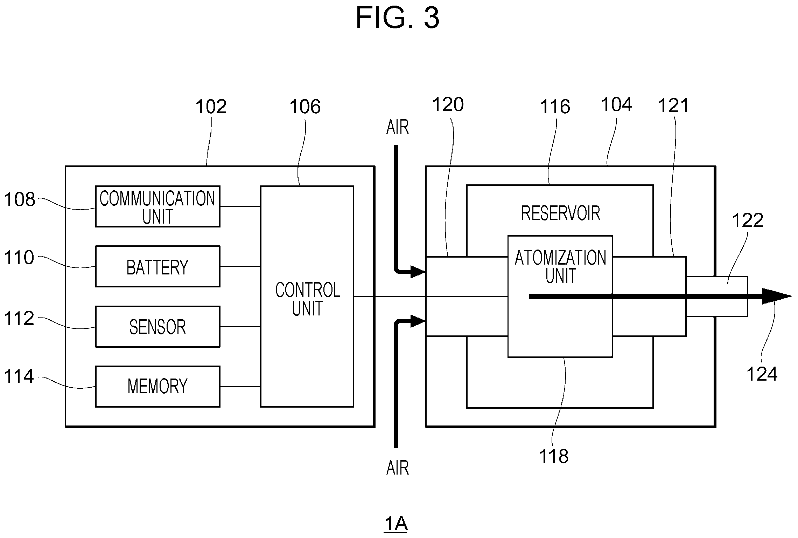

Fig. 3 is a block diagram illustrating a schematic configuration of the flavor inhaler according to the first embodiment of the present invention. As illustrated in Fig. 3, a flavor inhaler 1A includes a first member 102 and a second member 104. As illustrated in Fig. 3, for example, the first member 102 may include a control unit 106, a communication unit 108, a battery 110, a sensor 112, and a memory 114. The control unit 13 illustrated in Fig. 2A corresponds to the control unit 106 illustrated in Fig. 3, the sensor 11 illustrated in Fig. 2A corresponds to the sensor 112 illustrated in Fig. 3, and the communication unit 14 illustrated in Fig. 2A corresponds to the communication unit 108 illustrated in Fig. 3.

For example, the second member 104 may include a reservoir 116, an atomization unit 118, an air intake path 120, an aerosol path 121, and a mouthpiece unit 122. Some of the components included in the first member 102 may be included in the second member 104, instead. Some of the components included in the second member 104 may be included in the first member 102, instead. The second member 104 may be removably attached to the first member 102. Alternatively, all the components included in the first member 102 and the second member 104 may be included in the same case instead of the first member 102 and the second member 104.

The reservoir 116 holds an aerosol source. The reservoir 116 is composed of a fibrous or porous material, for example, and holds an aerosol source, which is a liquid, in gaps between fibers or pores in the porous material. Cotton, glass fiber, or a tobacco material, for example, may be used for the fibrous or porous material. The reservoir 116 may be configured as a tank that stores a liquid. The aerosol source is, for example, a liquid such as a polyhydric alcohol, which may be glycerin or propylene glycol, or water. When the flavor inhaler 1A is a medical inhaler such as a nebulizer, the aerosol source may include a medicine to be inhaled by a patient. In another example, the aerosol source may include a tobacco material or an extract derived from a tobacco material that releases a smoke flavor ingredient upon heating. The reservoir 116 may be configured such that a consumed aerosol source can be replenished. Alternatively, the reservoir 116 may be configured such that the reservoir 116 can be replaced after the aerosol source is consumed. The aerosol source is not limited to a liquid and may be a solid, instead. The reservoir 116 at a time when the aerosol source is a solid may be, for example, a hollow container that does not use a fibrous or porous material.

The atomization unit 118 is configured to generate an aerosol by atomizing the aerosol source. When the sensor 112 detects an inhalation, the atomization unit 118 generates an aerosol. A wick (not illustrated), for example, may be provided in such a way as to connect the reservoir 116 and the atomization unit 118 to each other. In this case, a part of the wick is inserted into the reservoir 116 and comes into contact with the aerosol source. Another part of the wick extends to the atomization unit 118. The aerosol source flows from the reservoir 116 to the atomization unit 118 due to a capillary effect produced by the wick. For example, the atomization unit 118 includes a heater electrically connected to the battery 110. The heater is disposed in such a way as to come into contact with or in close proximity to the wick. When an inhalation is detected, the control unit 106 controls the heater of the atomization unit 118 and atomizes the aerosol source that has flown through the wick by heating the aerosol source. Another example of the atomization unit 118 may be an ultrasonic atomizer that atomizes the aerosol source through ultrasonic vibration. The air intake path 120 is connected to the atomization unit 118 and leads to the outside of the flavor inhaler 1A. The aerosol generated by the atomization unit 118 mixes with air taken through the air intake path 120. A mixed fluid of the aerosol and the air flows into the aerosol path 121 as indicated by an arrow 124. The aerosol path 121 has a tubular structure for transporting the mixed fluid of the aerosol generated by the atomization unit 118 and the air to the mouthpiece unit 122.

The mouthpiece unit 122 is located at an end of the aerosol path 121 and configured to open the aerosol path 121 to the outside of the flavor inhaler 1A. The user puts the mouthpiece unit 122 in his/her mouth and inhales to take air including the aerosol into the oral cavity.

The communication unit 108 communicates with the user terminal or another computer. The communication unit 108 may be achieved by at least a network interface or the like as a hardware resource.

The battery 110 supplies power to some components of the flavor inhaler 1A, such as the communication unit 108, the sensor 112, the memory 114, and the atomization unit 118. The battery 110 may be charged by connecting the flavor inhaler 1A to an external power supply through a certain port (not illustrated). Only the battery 110 may be removed from the first member 102 or the flavor inhaler 1A and replaced by a new battery 110. The battery 110 may be replaced by a new battery 110 by replacing the entirety of the first member 102 with a new first member 102.

The sensor 112 may include a pressure sensor that detects variation in pressure inside the air intake path 120 and/or the aerosol path 121 or a flow rate sensor that detects a flow rate. The sensor 112 may also include a weight sensor that detects the weight of a component such as the reservoir 116. The sensor 112 may be configured to count the number of puffs by the user using the flavor inhaler 1A. The sensor 112 may be configured to accumulate periods of time for which the atomization unit 118 has been energized. The sensor 112 may also be configured to detect a liquid level inside the reservoir 116. The sensor 112 may also be configured to detect an SOC (state of charge), an integrated value of current, voltage, or the like of the battery 110. The integrated value of current may be obtained by current integration, an SOC-OCV (open circuit voltage) method, or the like. The sensor 112 may be an operation button that can be used by the user.

The control unit 106 may be an electronic circuit module configured as a microprocessor or a microcomputer, instead. The control unit 106 may be configured to control the operation of the flavor inhaler 1A in accordance with computer-executable instructions stored in the memory 114. The memory 114 is a storage medium such as a ROM, a RAM, or a flash memory. The memory 114 may also store setting data and the like necessary to control the flavor inhaler 1A, as well as the computer-executable instructions. For example, the memory 114 may store various pieces of data such as methods for controlling the communication unit 108 (modes of light emission, voice output, vibration, etc.), values detected by the sensor 112, and a heating history of the atomization unit 118. The control unit 106 reads data from the memory 114 as necessary, uses the data to control the flavor inhaler 1A, and stores the memory 114 as necessary.

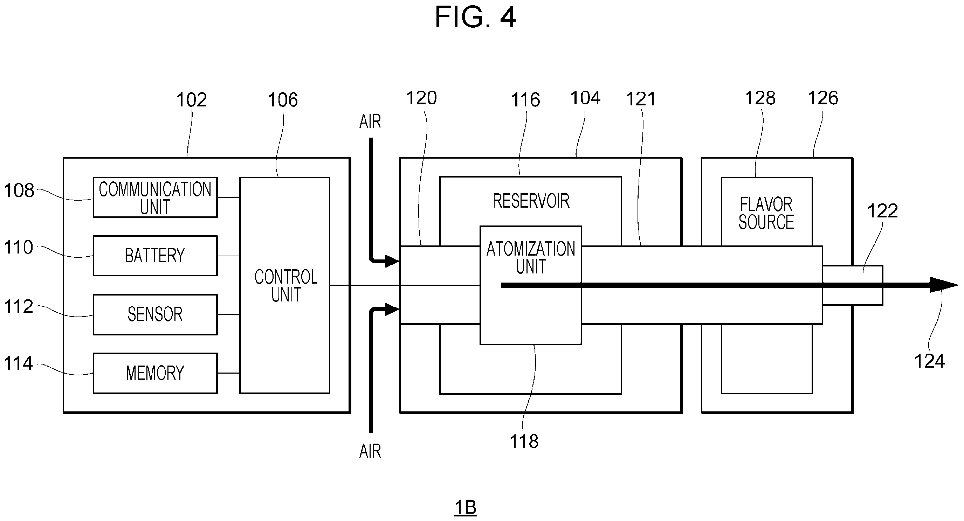

Fig. 4 is a block diagram illustrating another schematic configuration of the flavor inhaler according to the first embodiment of the present invention. As illustrated in Fig. 4, a flavor inhaler 1B includes a third member 126 in addition to the components included in the flavor inhaler 1A illustrated in Fig. 3. The third member 126 may include a flavor source 128. When the flavor inhaler 1B is an electronic cigarette, for example, the flavor source 128 may include a smoke flavor ingredient included in tobacco. As illustrated in Fig. 4, the aerosol path 121 extends across the second member 104 and the third member 126. The mouthpiece unit 122 is included in the third member 126.

The flavor source 128 is a component for giving a flavor to an aerosol. The flavor source 128 is disposed in the aerosol path 121. A mixed fluid of an aerosol generated by the atomization unit 118 and air (note that the mixed fluid might be simply referred to an aerosol hereinafter) flows to the mouthpiece unit 122 through the aerosol path 121. The flavor source 128 is thus provided downstream of the atomization unit 118 in terms of the flow of the aerosol. In other words, the flavor source 128 is closer to the mouthpiece unit 122 in the aerosol path 121 than the atomization unit 118 is. The aerosol generated by the atomization unit 118 therefore reaches the mouthpiece unit 122 after passing through the flavor source 128. When the aerosol passes through the flavor source 128, the smoke flavor ingredient included in the flavor source 128 is given to the aerosol. When the flavor inhaler 1B is an electronic cigarette, for example, the flavor source 128 may be fine-cut tobacco or a processed product derived from tobacco, such as a tobacco material molded into granules, sheets or powder. The flavor source 128 may be derived from a non-tobacco plant (e.g., mint, an herb, etc.), instead. For example, the flavor source 128 includes a tobacco ingredient. The flavor source 128 may contain a flavor ingredient such as menthol. As well as the flavor source 128, the reservoir 116 may also include a substance including a smoke flavor ingredient. For example, the flavor inhaler 1B may be configured to hold a flavor substance derived from tobacco in the flavor source 128 and include a flavor substance derived from non-tobacco plant in the reservoir 116.

The user can take air including the aerosol to which the flavor has been given into the oral cavity by inhaling through the mouthpiece unit 122.

The control unit 106 is configured to control the flavor inhalers 1A and 1B (hereinafter might be generically referred to as "flavor inhalers 1") according to the embodiment of the present disclosure.

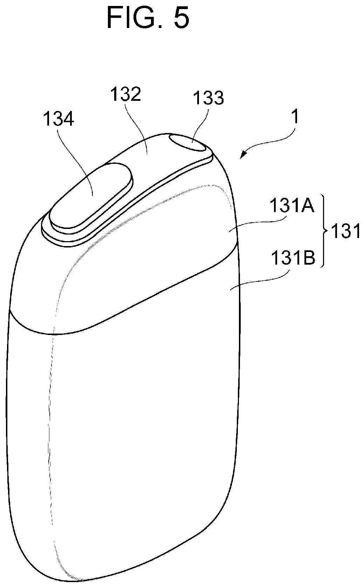

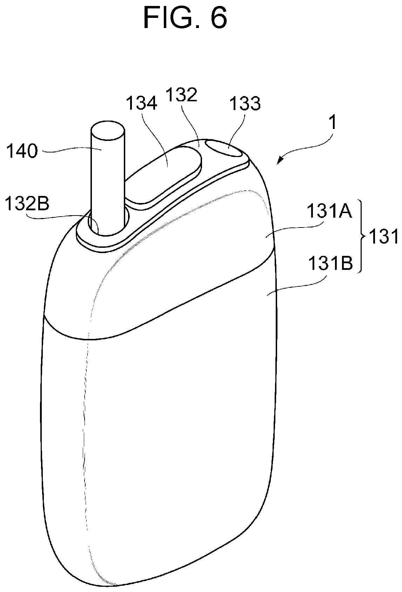

Fig. 5 is a diagram illustrating another example of the schematic appearance of the flavor inhaler according to the first embodiment of the present invention. Fig. 6 illustrates another example of the schematic appearance of the flavor inhaler at a time when the flavor inhaler holds an aerosol-source material according to the first embodiment of the present invention. In the present embodiment, for example, the flavor inhaler 1 is configured to generate an aerosol including a flavor by heating the aerosol-source material, such as a smoking article, including a flavor-source material such as a filler including an aerosol source and a flavor source. The smoking article 140 may be used as an aerosol-source material.

As those skilled in the art would understand, the smoking article 140 is just an example of the aerosol-source material. The aerosol source included in the aerosol-source material may be a solid or a liquid. The aerosol source may be, for example, a liquid such as a polyhydric alcohol, which may be glycerin or propylene glycol, or water. The aerosol source may include a tobacco material or an extract derived from a tobacco material that releases a smoke flavor ingredient upon heating. When the flavor inhaler 1 is a medical inhaler such as a nebulizer, the aerosol source may include a medicine to be inhaled by a patient. Depending on application, the aerosol-source material need not include a flavor source.

As illustrated in Figs. 5 and 6, the flavor inhaler 1 includes a top housing 131A, a bottom housing 131B, a cover 132, a switch 133, and a lid 134. The top housing 131A and the bottom housing 131B are connected to each other to form an outermost housing 131 of the flavor inhaler 1. The housing 131 may have a size that fits in a user's hand. In this case, when using the flavor inhaler 1, the user can inhale an aerosol while holding the flavor inhaler 1 by hand.

The top housing 131A has an opening (not illustrated), and the cover 132 is coupled with the top housing 131A in such a way as to cover the opening. As illustrated in Fig. 6, the cover 132 has an opening 132B into which the smoking article 140 can be inserted. The lid 134 is configured to open and close the opening 132B of the cover 132. More specifically, the lid 134 is attached to the cover 132 and configured to be able to move on a surface of the cover 132 between a first position, at which the opening 132B is closed, and a second position, at which the opening 132B is open.

The switch 133 is used to turn on and off the flavor inhaler 1. By using the switch 133 with the smoking article 140 inserted into the opening 132B as illustrated in Fig. 6, for example, the user can supply power to a heating unit (not illustrated) from a battery (not illustrated) and heat the smoking article 140 without burning the smoking article 140. When the smoking article 140 is heated, an aerosol is generated from the aerosol source included in the smoking article 140 and a flavor from the flavor source mixes with the aerosol. The user can inhale the aerosol including the flavor by inhaling through a part (a part illustrated in Fig. 6) of the smoking article 140 protruding from the flavor inhaler 1. A direction in which the aerosol-source material such as the smoking article 140 is inserted into the opening 132B will be referred to as a longitudinal direction of the flavor inhaler 1.

The configuration of the flavor inhaler 1 illustrated in Figs. 5 and 6 is just an example of the configuration of the flavor inhaler according to the present disclosure. The flavor inhaler according to the present disclosure can be configured in various modes in which an aerosol can be generated by heating the aerosol-source material including the aerosol source and the user can inhale the generated aerosol source.

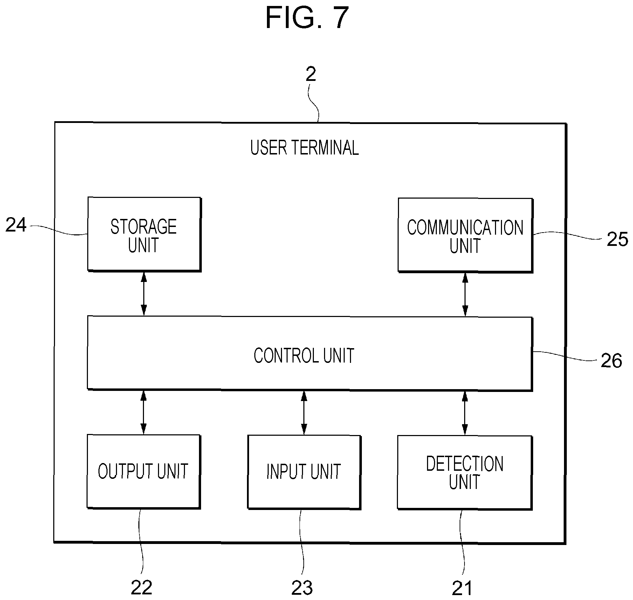

Fig. 7 is a block diagram illustrating a schematic configuration of the user terminal according to the first embodiment of the present invention. It is to be noted that Fig. 7 schematically and conceptually illustrates components included in the user terminal 2 and does not illustrate strict positions, shapes, dimensions, positional relationships, or the like of the components and the user terminal 2. It is also to be noted that the user terminal 2 can include a component that is not illustrated in Fig. 7. An example of the user terminal 2 is a single computer such as a smartphone, a tablet, or a personal computer, but is not limited to this. For example, a detection unit 21, which will be described hereinafter, may be achieved by a digital camera connected to a computer from the outside.

The detection unit 21 detects a certain change that occurs in at least a part of the flavor inhaler 1, that is, the change unit 12, for example, and that can be observed from the outside. The detection unit 21 may be achieved using at least a digital camera, a temperature sensor, or the like as a hardware resource.

An output unit 22 performs a process for outputting a message on the basis of output information sent from the control server 5 illustrated in Fig. 1. The output unit 22 displays information indicating that the certain change has been detected. The output unit 22 may be achieved using at least a display (includes a touch panel display) as a hardware resource.

An input unit 23 receives inputs from the user. The input unit 23 may be achieved using at least a keyboard and a mouse, a touch panel display, or the like as a hardware resource.

A storage unit 24 stores programs, data, and the like. The storage unit 24 may be achieved using at least an HDD (hard disk drive), an SSD (solid-state drive), a memory, or the like as a hardware resource.

A communication unit 25 communicates with other computers. The communication unit 25 may be achieved using at least a network interface or the like as a hardware resource.

A control unit 26 performs various types of control. The control unit 26 may be achieved using at least a processor or the like as a hardware resource. The control unit 26 may be configured to make a determination as to inhalation methods employed by the user. The output unit 22 can also display a result of the determination. The control unit 26 may be configured to allow the user to select one of the inhalation methods through the input unit 23. The output unit 22 can also display information based on a selected inhalation method. Furthermore, the control unit 26 may be configured to communicate data through the communication unit 25. The output unit 22 can also output information based on received data.

The various functions of the user terminal 2 may be achieved using an application that operates on the user terminal. The user terminal 2 may download the application and achieve the various functions using the downloaded application, instead. The user terminal 2 may achieve the various functions thereof by downloading a program for achieving the various functions and executing the downloaded program, instead.

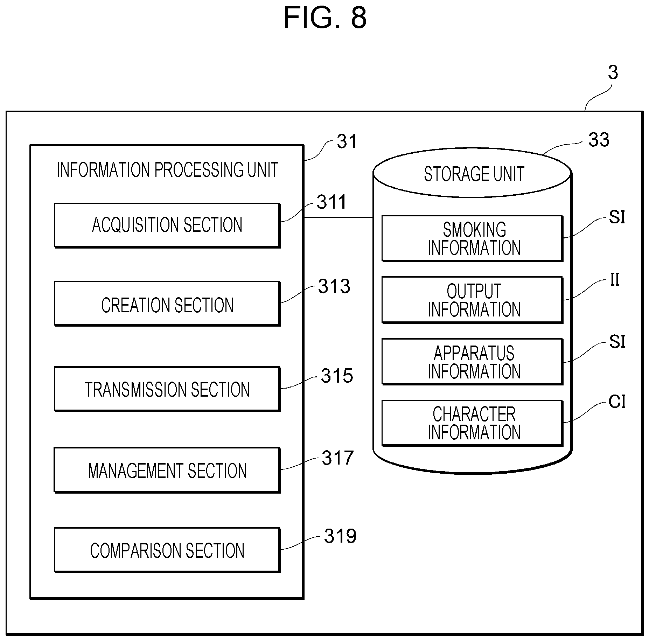

Fig. 8 is a schematic configuration diagram illustrating an example of the functional configuration of the information processing server according to the first embodiment of the present invention. As illustrated in Fig. 8, the information processing server 3 functionally includes an information processing unit 31 for performing a process for creating output information and a process for sending output information and a storage unit 33 that stores information for performing various types of processing, execution results of the various types of processing, and the like. The information processing server 3 is achieved, for example, by one or a plurality of server apparatuses. The information processing unit 31 can be achieved, for example, by executing a program (not illustrated) stored in the storage unit 33 using a CPU or the like. Alternatively, the information processing unit 31 may be achieved by downloading a program to be used for processing performed by the information processing unit 31 and executing the downloaded program.

The information processing unit 31 functionally includes an acquisition unit 311 that obtains smoking information from the flavor inhaler etc. 1 associated to the user terminal 2 illustrated in Fig. 1, a creation unit 313 that creates output information for outputting a message based on smoking information to the user terminal 2, a transmission unit 315 that sends output information to the user terminal 2 or the control server 5 that controls the output of messages to the user terminal 2, and a management unit 317 that manages one or a plurality of characters associated to one or a plurality of flavor inhalers etc. 1. The information processing unit 31 also includes a comparison unit 319 that compares use conditions of the plurality of flavor inhalers etc. 1 with one another on the basis of obtained smoking information. The comparison unit 319 achieves a function thereof in an output information provision system according to a second embodiment or a third embodiment including a plurality of flavor inhalers etc. 1.

As described above, the storage unit 33 stores smoking information SI, which is information regarding smoking, and output information II, which is created on the basis of the smoking information SI. The storage unit 33 also stores apparatus information SI regarding the flavor inhaler etc. 1 and character information CI regarding a character associated to each of one or a plurality of flavor inhalers etc. 1. The character information C1 may include, for example, information for forming an association with an icon image or audio information corresponding to each of characters held by the control server 5 or information for referring to the icon image or the audio information. The storage unit 33 may store the smoking information SI, the output information II, the apparatus information SI, and the character information CI with one another for each of users.

### [Process for Sending Output Information]

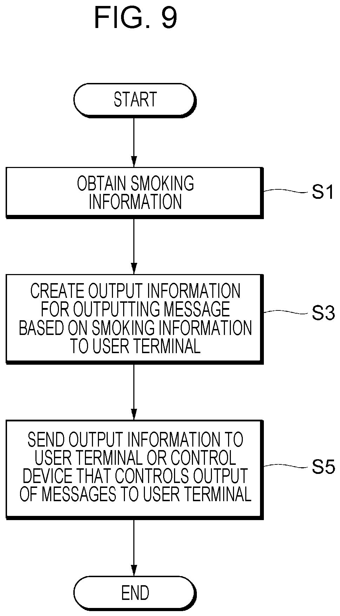

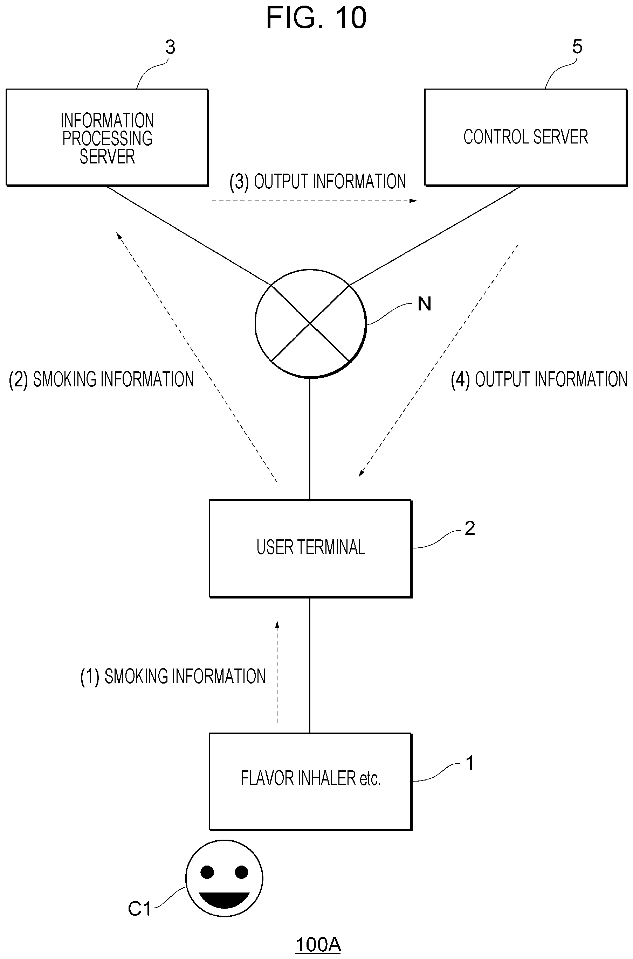

An example of the process for sending output information according to the first embodiment of the present invention will be described with reference to Figs. 9 and 10. Fig. 9 is a flowchart illustrating an example of the process for sending output information according to the first embodiment of the present invention. Fig. 10 is a conceptual diagram illustrating the example of the process for sending output information according to the first embodiment of the present invention.

### (Step S1 in Fig. 9)

The acquisition unit 311 of the information processing server 3 illustrated in Fig. 8 obtains smoking information from the flavor inhaler etc. 1 associated to the user terminal 2 illustrated in Fig. 1. As illustrated in Fig. 10, for example, the flavor inhaler etc. 1 sends smoking information to the user terminal 2 using BLE communication or the like ((1) smoking information in Fig. 10). The acquisition unit 311 then obtains the smoking information through the user terminal 2 ((2) smoking information in Fig. 10).

### (Step S3 in Fig. 9)

The creation unit 313 of the information processing server 3 illustrated in Fig. 8 creates output information for outputting a message based on the smoking information to the user terminal 2. For example, the creation unit 313 creates output information for outputting a message regarding at least a location of smoking or smoking time included in the smoking information obtained from the flavor inhaler etc. 1 to the user terminal 2 on the basis of information regarding the location of smoking or the smoking time. The smoking time may include information indicating a period of time for which the user has smoked as well as information indicating a time point. The creation unit 313 may calculate or estimate the amount of smoking from the smoking information and create output information for outputting a message regarding the calculated or estimate amount of smoking to the user terminal 2.

### (Step S5 in Fig. 9)

The transmission unit 315 of the information processing server 3 illustrated in Fig. 8 sends output information to the control server 5 that manages the information exchange system capable of sending output information to the user terminal 2 ((3) output information in Fig. 10). For example, the transmission unit 315 may send character information regarding a character to the user terminal 2 along with the output information so that the output information is output on the user terminal 2 as a message while being associated to the character information. The control server 5 transfers, to the user terminal 2, the output information received from the information processing server 3 and image information or audio information regarding the character corresponding to character information ((4) output information in Fig. 10). The character information may be information included in the output information, instead. The user terminal 2 then outputs a message with the output unit 22 illustrated in Fig. 7 on the basis of the image information or audio information regarding the character. The transmission unit 315 may transfer the output information and the image information or audio information regarding the character corresponding to the character information to the user terminal 2 at any time. For example, the transmission unit 315 may transfer these pieces of information to the user terminal 2 at a time when smoking information is obtained from the flavor inhaler etc. 1. Alternatively, the transmission unit 315 may transfer these pieces of information to the user terminal 2 at a preset time, namely, for example, 10 p.m. Alternatively, the transmission unit 315 may transfer these pieces of information to the user terminal 2 at certain time intervals, namely, for example, every day or every week.

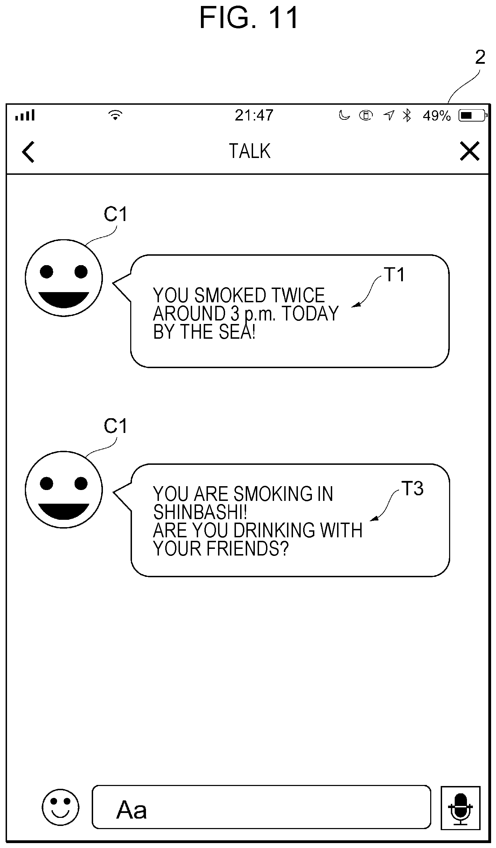

Fig. 11 is a diagram illustrating an example of a screen output on the user terminal according to the first embodiment of the present invention. As illustrated in Fig. 11, for example, text information is output on the user terminal 2 as a message while being associated to a character icon image C1 (refer to Fig. 10) regarding a character associated to the flavor inhaler etc. 1. For example, a text T1 at least includes information regarding smoking time "around 3 p.m. today" and information regarding a location of smoking "by the sea". The text T1 also includes information regarding the amount of smoking "twice". A text T3 includes information regarding a location of smoking "Shinbashi".

The character icon image C1 may make a certain motion therein. For example, the icon image C1 may make a motion corresponding to an emotion or a motion such as a vertical or horizontal movement or rotation. The character icon image C1 may make various motions on the basis of details or attributes of a character therein. In the case of a character that imitates a person, an animal, or the like, for example, the motion made by the character icon image C 1 may be a motion made by a person, an animal, or the like, such as movement of limbs, walking, running, or jumping. In the case of a character that imitates an automobile, an airplane, or the like, the motion made by the character icon image C1 may be a motion made by an automobile, an airplane, or the like, such as driving or flying. The motion made by the character icon image C1 is not limited to these motions and may be any kind of motion. Alternatively, the motion made by the character icon image C1 may be determined on the basis of content of the text T1. If the content of the text T1 has a negative connotation, for example, the motion made by the character icon image C1 may include a negative motion such as crying or grieving. If the content of the text T1 has a positive connotation, on the other hand, the motion made by the character icon image C1 may be a negative motion such as laughing and showing of joy. The motion made by the character icon image C1 is not limited to these examples and may be any kind of motion.

With this configuration, the user can have a conversation with a character associated to the flavor inhaler etc. 1 on a screen on which output information is output. Because, through the character, the user is attached to the flavor inhaler etc. 1 associated to the character, the retention rate for users using the flavor inhaler etc. 1 can be increased.

The message may include audio information regarding the character associated to the flavor inhaler etc. 1 while associating the audio information with the character icon image C1 (refer to Fig. 10) regarding the character. That is, the user terminal 2 may output a sound corresponding to the character as a message. A tone of the sound corresponding to the character may change on the basis of the content of the text T1. If the content of the text T1 has a negative connotation, for example, the sound corresponding to the character may have a sad tone. If the content of the text T1 has a positive connotation, on the other hand, the sound corresponding to the character may have a happy tone. The speed or an interval of the sound corresponding to the character may change on the basis of the content of the text T1. The character may correspond to a certain person, and in this case, the sound corresponding to the character may be a voice of the certain person.

With this configuration, a conversation with the character associated to the flavor inhaler etc. 1 can be performed with not only text information and image information but also a unique voice of the character.

The screen on which output information is output need not display a character. In this case, the screen on which the output information is output may display only text information. With this configuration, the user can receive the text information by using the flavor inhaler etc. 1 and be attracted to the flavor inhaler etc. 1 to a certain degree.

The screen on which the output information is output may display only a character, instead. In this case, the message is output as audio information regarding the character. With this configuration, the user can have a conversation with a character associated to the flavor inhaler etc. 1 using a unique voice of the character by using the flavor inhaler etc. 1.

The message may include movie information relating to the character. In this case, the user terminal 2 displays a movie such as an animation as a message, and the character may issue the message in the movie. When the character corresponds to a certain person, the user terminal 2 may display a video message in which the certain person appears, and the certain person may issue a message in the video message. The movie information included in the message is not limited to these examples and may be any kind of movie information. With this configuration, the user can have a conversation with a character associated to the flavor inhaler etc. 1 using a movie relating to the character.

The message may be configured to be deleted from the user terminal 2 on the basis of a fact that the message has been displayed on the user terminal 2 for a certain period of time. Alternatively, the message may be configured to be deleted from the user terminal 2 in accordance with a request from the user of the user terminal 2. Alternatively, the message may be configured to be deleted from the user terminal 2 on the basis of detection of a certain action. With this configuration, a message including content relating to smoking information is deleted from the user terminal 2 when a certain period of time has elapsed or a request from the user or the certain action is detected, the confidentiality of specific content relating to the smoking information can be secured.

The message may include a URL (uniform resource locator), a QR code (registered trademark), or the like relating to a certain website on the Internet. In this case, the user may be able to view the certain website on which text information, a character icon image, or the like is displayed by reading the URL or the QR code on the user terminal 2. Alternatively, authentication employing a password or the like may be performed before the user views the certain website using the user terminal 2. With this configuration, text information or character icon information can be viewed on a certain website. Because only a URL or a QR code is displayed in a message, the confidentiality of specific content relating to smoking information can be secured.

The message may include image information or movie information displayed when a certain operation performed by the user is detected. The certain operation performed by the user is, for example, inputting of a password to the user terminal 2, an inhalation using the flavor inhaler etc. 1, or the like. With this configuration, text information or character icon information can be viewed on a certain website. Because only a URL or a QR code is displayed in a message, the confidentiality of specific content relating to smoking information can be secured.

The message may include image information or movie information in which text information can be scrolled in vertical and/or horizontal directions. For example, text information displayed on the user terminal 2 may be configured to be scrolled from a right edge of a screen to a left edge. With this configuration, since the text information is scrolled and displayed on the user terminal 2 only for a certain period of time, the confidentiality of specific content relating to smoking information can be secured.

If the creation unit 313 does not obtain smoking information from the flavor inhaler etc. 1 for a certain period of time (e.g., several days), output information for outputting, to the user terminal 2, a message based on the fact that smoking information has not been obtained for the certain period of time may be created. For example, the message may be text information such as "Information regarding smoking has not recently been obtained", audio information, or movie information.

With this configuration, the user terminal 2 outputs output information for outputting, to the user terminal 2, a message based on a fact that smoking information has not been obtained. The user, therefore, can be notified of, for example, a possibility that the flavor inhaler etc. 1 has not been used for a certain period of time or a possibility of a lost or broken flavor inhaler etc. 1 or the like.

The user terminal 2 may display, using a dedicated or general-purpose application, a screen on which output information is output. The user terminal 2 may download the dedicated or general-purpose application and display, using the downloaded application, the screen on which the output information is output. Alternatively, the user terminal 2 may display the display information by downloading a program for displaying the screen on which the output information is output and executing the downloaded program. Alternatively, the user terminal 2 may achieve, using a function of a dedicated or general-purpose web browser, the displaying of the screen on which the output information is output.

According to the first embodiment of the present invention described above, output information for outputting a message based on smoking information from a flavor inhaler etc. to a user terminal is created and sent to the user terminal. The output information, therefore, can be appropriately provided in accordance with a smoking state of the user, and the retention rate for users using the flavor inhaler etc. can be increased.

### <Second Embodiment>

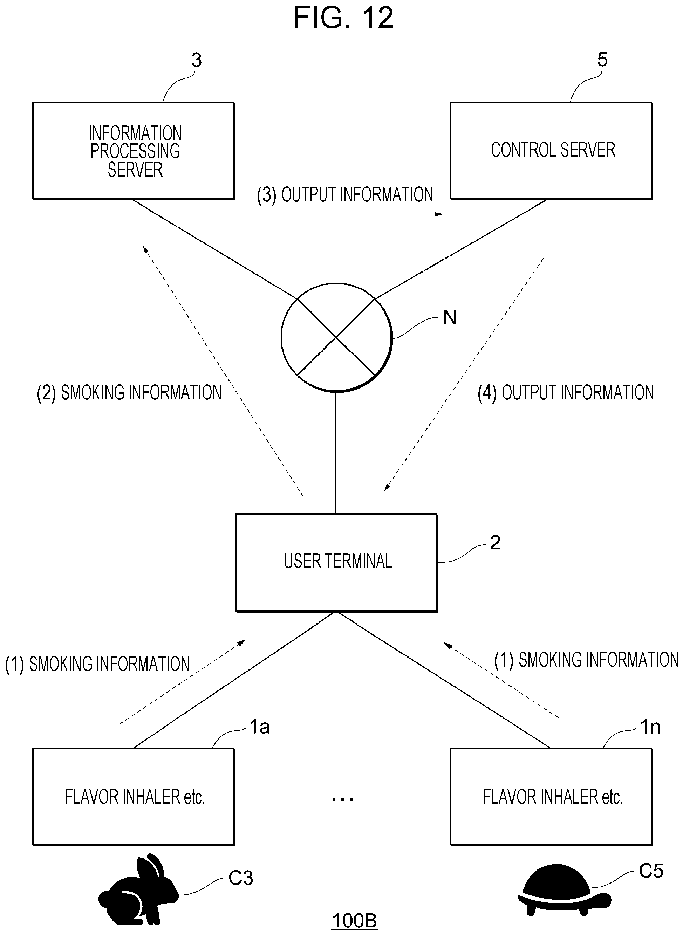

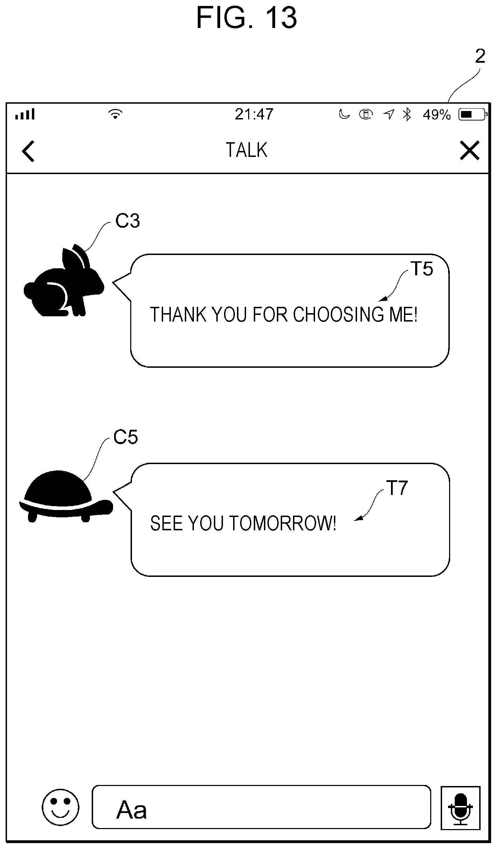

An output information provision system 100B according to a second embodiment of the present invention will be described with reference to Figs. 12 and 13. Fig. 12 is a schematic configuration diagram of the output information provision system according to the second embodiment of the present invention. As illustrated in Fig. 12, the output information provision system 100B according to the second embodiment is different from the output information provision system 100A according to the first embodiment in that the output information provision system 100B includes a single user terminal 2 and a plurality of flavor inhalers etc. 1 are associated to the single user terminal 2. Differences from the output information provision system 100A according to the first embodiment will be particularly described hereinafter. Fig. 12 illustrates a flavor inhaler etc. 1a and a flavor inhaler etc. In as n flavor inhalers etc. When these n flavor inhalers etc. are not distinguished from one another in the following description, the n flavor inhalers etc. will be simply referred to as "flavor inhalers etc. 1" while omitting part of reference numerals.

As illustrated in Fig. 12, the flavor inhalers etc. 1a and 1n send smoking information to the user terminal 2 using BLE communication or the like ((1) smoking information in Fig. 12). The information processing server 3 then obtains the smoking information through the user terminal 2 ((2) smoking information in Fig. 12). The transmission unit 315 illustrated in Fig. 8 sends output information and character information to the control server 5 ((3) output information in Fig. 12). The control server 5 refers to the character information and sends a character icon image C3 (a hare icon) and a character icon image C5 (a tortoise icon) regarding characters associated to the flavor inhalers etc. 1a and In, respectively, to the user terminal 2 along with the output information associated to the flavor inhalers etc. 1a and 1n so that the output information is output on the user terminal 2 as messages while being associated to the character icon images C3 and C5 ((4) output information in Fig. 12).

Fig. 13 is a diagram illustrating an example of a screen output on the user terminal according to the second embodiment of the present invention. As illustrated in Fig. 13, a text T5 associated to the flavor inhaler etc. 1a is output as a message while being associated to the character icon image C3 regarding the character associated to the flavor inhaler etc. 1a illustrated in Fig. 12. A text T7 associated to the flavor inhaler etc. In is output as a message while being associated to the character icon image C5 regarding the character associated to the flavor inhaler etc. In illustrated in Fig. 12. As with the character icon image C1 according to the first embodiment of the present invention, the character icon image C3 and the character icon image C5 may make certain motions therein.

With this configuration, output information associated to each of a plurality of flavor inhalers etc. is output as a message while being associated to character information regarding a character associated to the flavor inhaler etc. The user, therefore, can have a conversation with the character associated to each of the plurality of flavor inhalers etc. through a screen on which the message is output. Because, through the character, the user is attached to each of the flavor inhalers etc. associated to the characters, the retention rate for users using the plurality of flavor inhalers etc. can be increased. The screen on which the output information is output need not display a character. In this case, the screen on which the output information is output may display only text information. With this configuration, the user can receive the text information using the plurality of flavor inhalers etc. 1 and be attached to the plurality of flavor inhalers etc. 1 to a certain degree.

The comparison unit 319 of the information processing server 3 illustrated in Fig. 8 compares use conditions of the flavor inhalers etc. 1a and 1n with each other on the basis of obtained smoking information. The creation unit 313 creates output information for outputting, to the user terminal 2, the messages based on the smoking information obtained from the flavor inhalers etc. 1a and In. For example, the creation unit 313 creates the output information for outputting the message to the user terminal 2 in accordance with the use conditions on the basis of a result of the comparison performed by the comparison unit 319. For example, the comparison unit 319 illustrated in Fig. 8 identifies the flavor inhaler etc. 1a whose use frequency is higher and the flavor inhaler etc. In whose use frequency is lower, and the creation unit 313 creates output information corresponding to the flavor inhaler etc. 1a, whose use frequency is higher, and output information corresponding to the flavor inhaler etc. In, whose use frequency is lower, on the basis of the result of comparison such that these pieces of output information become different from each other. In the example illustrated in Fig. 13, a message corresponding to the flavor inhaler etc. 1a, whose use frequency is higher, is a text T5, "Thank you for choosing me!", and a message corresponding to the flavor inhaler etc. In, whose use frequency is lower, is a text T7, "See you tomorrow! "

With this configuration, a message corresponding to a flavor inhaler etc. whose use frequency is higher and a message corresponding to a flavor inhaler etc. whose use frequency is lower are different from each other when being output, and the user can accurately and easily understand which of the flavor inhalers etc. he/she has used most frequently in a certain period.

As in the first embodiment of the present invention, the messages may include audio information regarding characters, movie information relating to the characters, or the like.

The creation unit 313 of the server 3 may create output information for outputting, to the user terminal 2, information regarding a result of comparison performed by the comparison unit 319 as well as messages based on smoking information. The information regarding a result of comparison is, for example, comprehensive information for notifying the user of the result of comparison, such as a graph (e.g., a pie chart) indicating a proportion of the amount of smoking of each of the flavor inhalers etc. to the total amount of smoking by the user or text information indicating the proportion in percentage. With this configuration, information regarding a result of comparison between a flavor inhaler etc. whose use frequency is higher and a flavor inhaler etc. whose use frequency is lower is output, and the user can accurately and easily understand which of the flavor inhalers etc. the user has used most frequently in a certain period.

According to the second embodiment of the present invention described above, in the output information provision system 100B that includes the single user terminal 2 and in which a plurality of flavor inhalers etc. 1 are associated to the single user terminal 2, too, output information can be appropriately provided in accordance with a smoking state of the user who uses the plurality of flavor inhalers etc. 1, and the retention rate for users using the plurality of flavor inhalers etc. 1 can be increased.

### <Third Embodiment>

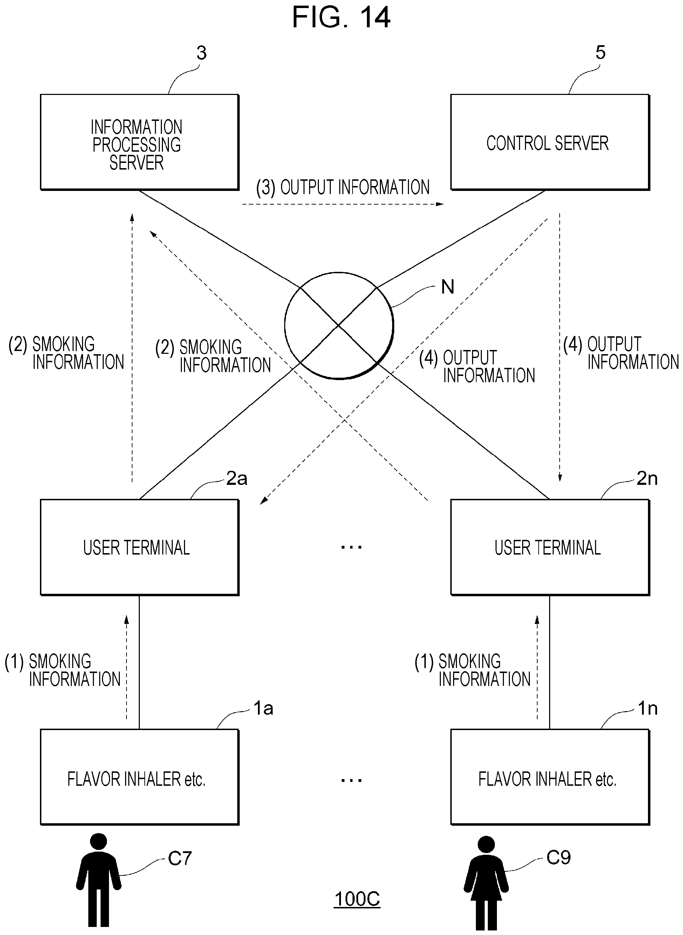

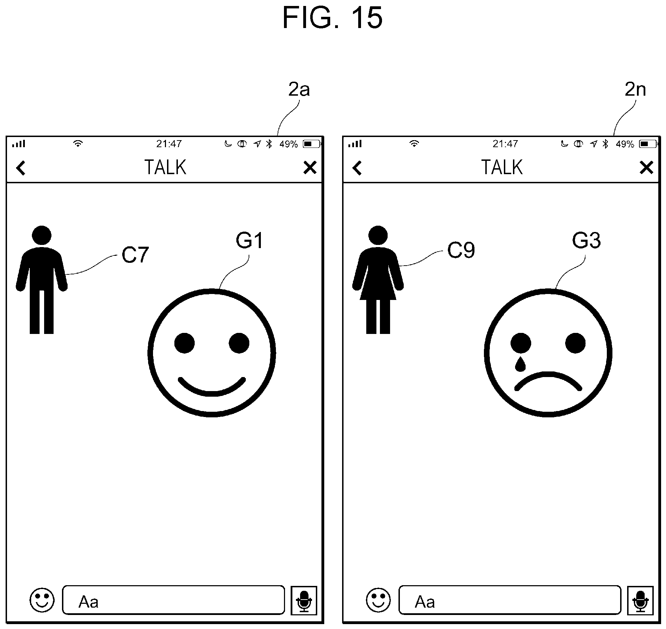

An output information provision system 100C according to a third embodiment of the present invention will be described with reference to Figs. 14 and 15. Fig. 14 is a schematic configuration diagram illustrating the output information provision system according to the third embodiment of the present invention. As illustrated in Fig. 14, the output information provision system 100C according to the third embodiment is different from the output information provision system 100A according to the first embodiment in that the output information provision system 100C includes a plurality of user terminals 2 and a flavor inhaler etc. 1 is associated to each of the plurality of user terminals 2. Differences from the output information provision system 100A according to the first embodiment will be particularly described hereinafter. Fig. 14 illustrates a user terminal 2a and a user terminal 2n as n user terminals. When the n user terminals are not distinguished from one another in the following description, the n user terminals will be simply referred to as "user terminals 2" while omitting part of reference numerals.

As illustrated in Fig. 10, each of the flavor inhalers etc. 1a and 1n sends smoking information to a different one of the plurality of user terminals 2a and 2n using BLE communication or the like ((1) smoking information in Fig. 14). The information processing server 3 then obtains smoking information through the plurality of user terminals 2a and 2n ((2) smoking information in Fig. 14). The transmission unit 315 illustrated in Fig. 8 sends output information and character information to the control server 5 ((3) output information in Fig. 14). The control server 5 sends a character icon image C7 (a male icon) or a character icon image C9 (a female icon) regarding a character associated to each of the flavor inhalers etc. 1a and 1n to a corresponding one of the plurality of user terminals 2 along with the output information associated to the flavor inhaler etc. 1a or 1n so that the output information is output on the user terminal 2 as a message while being associated to the character icon image C7 or C9 ((4) output information in Fig. 14). As with the character icon image C1 according to the first embodiment of the present invention, the character icon image C7 and the character icon image C9 may make certain motions therein.

Fig. 15 is a diagram illustrating an example of screens output on the user terminals according to the third embodiment of the present invention. The comparison unit 319 of the information processing server 3 illustrated in Fig. 8 compares use conditions of the plurality of flavor inhalers etc. 1a and 1n with each other on the basis of the obtained smoking information. The creation unit 313 creates output information for outputting, to the user terminals 2, messages based on the smoking information obtained from the plurality of flavor inhalers etc. 1a and 1n. For example, the creation unit 313 creates the output information for outputting a message to at least one of the user terminals 2 in accordance with the use conditions on the basis of a result of the comparison performed by the comparison unit 319. For example, the comparison unit 319 illustrated in Fig. 8 identifies, on the basis of the obtained smoking information, the flavor inhaler etc. 1a used by a user whose amount of smoking is larger and a flavor inhaler etc. In used by a user whose amount of smoking is smaller, and the creation unit 313 creates output information corresponding to the flavor inhaler etc. 1a, which is used by the user whose amount of smoking is larger, and output information corresponding to the flavor inhaler etc. 1n, which is used by the user whose amount of smoking is smaller, on the basis of the result of comparison such that these pieces of output information become different from each other. As illustrated in Fig. 15, for example, the output information corresponding to the flavor inhaler etc. 1a, which is used by the user whose amount of smoking is larger, is a character face image G1 indicating a smile (joy), and the output information corresponding to the flavor inhaler etc. In, which is used by the user whose amount of smoking is smaller, is a character face image G3 indicating a crying face (sadness). The output information may include a character image with which an emotion can be identified. For example, the character image may be a character face image indicating one of various emotions typified by joy, anger, sorrow, and pleasure.

With this configuration, a message corresponding a flavor inhaler etc. used by a user whose amount of smoking is larger and a message corresponding to a flavor inhaler etc. used by a user whose amount of smoking is smaller are different from each other when being output. The user, therefore, can accurately and easily understand which of the flavor inhalers etc. has been used for smoking more frequently or less frequently in a certain period.

As illustrated in Fig. 15, the character face image G1 associated to the flavor inhaler etc. 1a is output as a message while being associated to the character icon image C7, which is illustrated in Fig. 14, regarding the character associated to the flavor inhaler etc. 1a. The character face image G3 associated to the flavor inhaler etc. In is output as a message while being associated to the character icon image C9, which is illustrated in Fig. 14, regarding the character associated to the flavor inhaler etc. In.

With this configuration, output information associated to each of the plurality of flavor inhalers etc. is output as a message while being associated to character information regarding a character associated to the flavor inhaler etc. Each of users can therefore have a conversation with the character associated to one of the flavor inhalers etc. associated to the user through a screen on which the output information is output. Because, through the character, the user is attached to the flavor inhaler etc. associated to the character, the retention rate for users using the flavor inhaler etc. can be increased.

As in the first embodiment of the present invention, the message may include audio information regarding a character, movie information relating to a character, or the like.

The creation unit 313 of the information processing server 3 may also create output information for outputting, to the user terminals 2, information regarding a result of comparison performed by the comparison unit 319 as well as messages based on smoking information. The information regarding a result of comparison is, for example, comprehensive information for notifying each of the users of the result of comparison of the amount of smoking by the users. With this configuration, since the information regarding the result of comparison of the amount of smoking by the user and the amount of smoking by the other users with each other is output for the user, the user can accurately and easily understand which of the users has frequently used the flavor inhaler etc. in a certain period.

With the third embodiment of the present invention described above, in the output information provision system 100C that includes a plurality of user terminals 2 and in which a flavor inhaler etc. 1 is associated to each of the plurality of user terminals 2, too, output information can be appropriately provided in accordance with smoking states of the plurality of users who use the flavor inhalers etc. 1, and the retention rate for each of the plurality of users using the flavor inhalers etc. 1 can be increased.

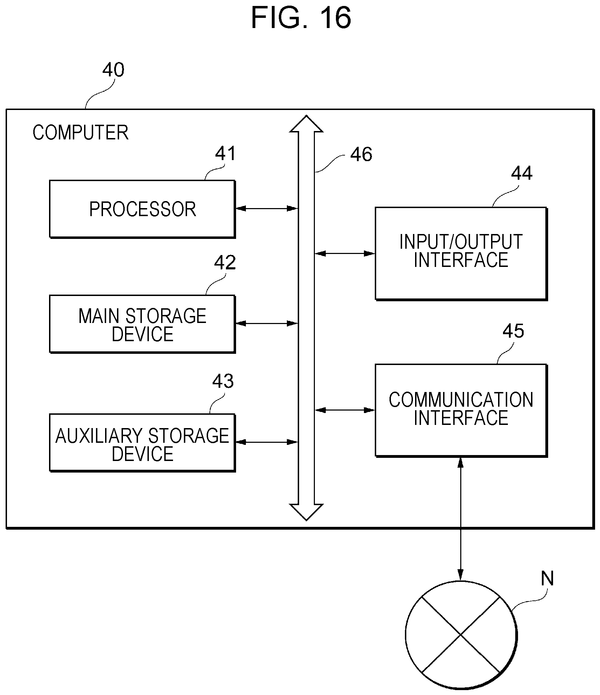

Fig. 16 is a diagram illustrating an example of the hardware configuration of a computer according to an embodiment of the present invention. An example of the hardware configuration of a computer that can be used as the user terminal 2, the information processing server 3, or the control server 5 illustrated in Fig. 1 or that can be used to achieve the user terminal 2, the information processing server 3, or the control server 5 will be described with reference to Fig. 16.

As illustrated in Fig. 16, a computer 40 mainly includes, as hardware resources, a processor 41, a main storage device 42, an auxiliary storage device 43, an input/output interface 44, and a communication interface 45, and these components are connected to one another through a bus line 46 including an address bus, a data bus, a control bus, and the like. An interface circuit (not illustrated) might exist between the bus line 46 and each of the hardware resources.

The processor 41 controls the entirety of the computer. The main storage device 42 provides a working area for the processor 41 and is a nonvolatile memory such as an SRAM (static random-access memory) or a DRAM (dynamic random-access memory). The auxiliary storage device 43 is a nonvolatile memory storing programs, data, and the like, which are software, such as an HDD, an SSD, or a flash memory. The programs, the data, and the like are loaded into the main storage device 42 from the auxiliary storage device 43 through the bus line 46 at any point in time.

The input/output interface 44 provides and/or receives information and is a digital camera, a keyboard, a mouse, a display, a touch panel display, a microphone, a speaker, a temperature sensor, and/or the like. The communication interface 45 is connected to a network N and communicates data through the network N. The communication interface 45 and the network N can be connected by wire or wirelessly. The communication interface 45 can obtain information regarding the network, namely, for example, information regarding an access point of Wi-Fi or information regarding a base station of a communication carrier.

It is obvious to those skilled in the art that, as a result of cooperation of the above-described hardware resources and the software, the computer 40 can function as desired means, perform desired steps, and achieve desired functions.

The above embodiments are provided in order to facilitate understanding of the present invention and not intended to limit interpretation of the present invention. The present invention can be modified or improved without deviating from the scope thereof and also includes equivalents thereof.

### Reference Signs List

- 1: flavor inhaler etc.
- 2: user terminal
- 3: information processing server
- 5: control server
- 11: sensor
- 12: change unit
- 13: control unit
- 21: detection unit
- 22: output unit
- 23: input unit
- 24: storage unit
- 25: communication unit
- 26: control unit
- 31: information processing unit
- 33: storage unit
- 100A, 100B, 100C: output information provision system
- 161, 162: end
- 311: acquisition unit
- 313: creation unit
- 315: transmission unit
- 317: management unit
- 319: comparison unit

## Claims

1. An information processing device comprising:
an acquisition unit that obtains smoking information from a flavor inhaler associated to a user terminal;
a creation unit that creates output information for outputting a message based on the smoking information, to the user terminal; and
a transmission unit that sends the output information to the user terminal or a control device that controls output of messages to the user terminal.

2. The information processing device according to Claim 1,
wherein the creation unit creates the output information for outputting, to the user terminal, the message regarding at least a location of smoking or smoking time on a basis of information regarding the location of smoking or the smoking time included in the smoking information.

3. The information processing device according to Claim 1 or 2,
wherein, if the smoking information is not obtained from the flavor inhaler within a certain period, the creation unit creates the output information for outputting, to the user terminal, the message based on a fact that the smoking information has not been obtained within the certain period.

4. The information processing device according to any of Claims 1 to 3, further comprising:
a managing unit that manages a character associated to the flavor inhaler,
wherein the acquisition unit obtains apparatus information regarding the flavor inhaler, and
wherein the message includes image information regarding the character associated to the apparatus information.

5. The information processing device according to any of Claims 1 to 4, further comprising:
a management unit that manages a character associated to the flavor inhaler,
wherein the acquisition unit obtains apparatus information regarding the flavor inhaler, and
wherein the message includes audio information regarding the character associated to the apparatus information.

6. The information processing device according to any of Claims 1 to 5, further comprising:
a management unit that manages a character associated to the flavor inhaler,
wherein the acquisition unit obtains apparatus information regarding the flavor inhaler, and
wherein the message includes movie information regarding the character associated to the apparatus information.

7. The information processing device according to Claim 1,
wherein the acquisition unit obtains smoking information from each of a plurality of flavor inhalers associated to the user terminal, and
wherein the creation unit creates the output information for outputting, to the user terminal, the message based on the smoking information obtained from each of the plurality of flavor inhalers.

8. The information processing device according to Claim 7, further comprising:
a comparison unit that compares use conditions of the plurality of flavor inhalers with one another on a basis of the obtained smoking information,
wherein the creation unit creates output information corresponding to a flavor inhaler whose use frequency is higher and output information corresponding to a flavor inhaler whose use frequency is lower on a basis of a result of the comparison performed by the comparison unit such that these pieces of output information become different from each other.

9. The information processing device according to Claim 1,
wherein the acquisition unit obtains smoking information from each of a plurality of flavor inhalers associated to a different one of a plurality of user terminals, and
wherein the creation unit creates the output information for outputting, to at least one of the plurality of user terminals, the message based on the smoking information obtained from each of the plurality of flavor inhalers.

10. The information processing device according to Claim 9, further comprising:
a comparison unit that compares use conditions of the plurality of flavor inhalers with one another on a basis of the obtained smoking information,
wherein the creation unit creates output information corresponding to a flavor inhaler used by a user whose amount of smoking is larger and output information corresponding to a flavor inhaler used by a user whose amount of smoking is smaller on a basis of a result of the comparison performed by the comparison unit such that these pieces of output information become different from each other.

11. The information processing device according to any of Claims 1 to 10,
wherein the message includes image information regarding a character with which an emotion can be identified.

12. An information processing method executed by a computer, the information processing method comprising the steps of:
obtaining smoking information from a flavor inhaler associated to a user terminal;
creating output information for outputting a message based on the smoking information to the user terminal; and
sending the output information to the user terminal or a control device that controls output of messages to the user terminal.

13. A program for causing a computer to achieve:
an acquisition unit that obtains smoking information from a flavor inhaler associated to a user terminal;
a creation unit that creates output information for outputting a message based on the smoking information, to the user terminal; and
a transmission unit that sends the output information to the user terminal or a control device that controls output of messages to the user terminal.
